# EUROPÄISCHE PATENTSCHRIFT

(11) **EP 2 601 179 B1**
(45) Veröffentlichungstag und Bekanntmachung des Hinweises auf die Patenterteilung: **19.10.2016**
(21) Anmeldenummer: 11733835.0
(22) Anmeldetag: 14.07.2011
(51) Int. Cl.: C08G 65/26, C07D 251/70

(54) **VERFAHREN ZUR HERSTELLUNG VON AMINOTRIAZIN-ALKOXILATEN**
PROCESS FOR PREPARING AMINOTRIAZINE ALKOXYLATES
PROCÉDÉ POUR LA PRÉPARATION D'ALCOXYLATE D'AMINOTRIAZINE

(30) Priorität: 05.08.2010 EP 10172024
(43) Veröffentlichungstag der Anmeldung: 12.06.2013
(73) Patentinhaber: BASF SE, 67056 Ludwigshafen am Rhein (DE)
(72) Erfinder: KUNST, Andreas, 67063 Ludwigshafen (DE); GARNIER, Sebastien, 12161 Berlin (DE); GEHRINGER, Lionel, F-67470 Schaffhouse-pres-Seltz (FR); LÖFFLER, Achim, 67346 Speyer (DE)
(74) Vertreter: BASF IP Association
(86) Internationale Anmeldenummer: PCT/EP2011/062021
(87) Internationale Veröffentlichungsnummer: WO 2012/016803

(56) Entgegenhaltungen:
- EP-A1- 0 051 734
- WO-A2-2009/144274
- US-A- 4 356 304

## Beschreibung

Die vorliegende Erfindung betrifft ein Verfahren zur Herstellung von Amino-1,3,5-triazin-Polyetherolen (im Folgenden als Aminotriazin-Alkoxilate bezeichnet), insbesondere von 2,4,6-Triamino-1,3,5-triazin-Polyetherolen (auch Melamin-Alkoxilate genannt), sowie die erfindungsgemäß herstellbaren Amino-1,3,5-triazin-Polyetherole und deren Verwendungen.

### Stand der Technik:

Die Herstellung von alkoxilierten Aminotriazinen ist prinzipiell bekannt. US 3812122 beschreibt ein Verfahren, bei dem Aminotriazine in Gegenwart von basischen Katalysatoren in N,N-Dialkylcarbonsäureamiden als Lösungsmittel bei Temperaturen von 90 - 200°C mit Alkylenoxiden zur Reaktion gebracht werden.

US 3328321 beschreibt, dass Melamin-Alkoxilate z. B. durch Reaktion von Melamin mit Ethylencarbonat oder Alkylenoxiden in Gegenwart von basischen Katalysatoren und Lösungsmitteln wie Diethylenglykoldimethylether, Dimethylformamid u. a. hergestellt werden können.

US 3438986 beschreibt ein Verfahren zur Herstellung von Aminotriazin-Alkoxilaten durch Umsetzung von Aminotriazinen mit Alkylenoxiden in Gegenwart von Aryldiaminen als Lösungsmittel.

In GB 1064148 ist beschrieben worden, dass die Umsetzung von Aminotriazinen unter basischen Bedingungen mit Alkylenoxiden auch in Gegenwart von aromatischen, gesättigten oder ungesättigten Polyolen erfolgen kann. Die Verwendung von Melamin jedoch erfordert die zusätzliche Anwesenheit eines inerten Lösungsmittels wie Dimethylsulfoxid.

DE 3412082A1 beschreibt die Umsetzung von Aminotriazinen mit Alkylenoxiden ohne die Anwesenheit eines Katalysator und ohne die Verwendung eines inerten Lösungsmittels. Jedoch wird bei diesem Verfahren mindestens ein 2-6-wertiger aliphatischer und/oder cycloaliphatischer Alkohol als Costarter eingesetzt.

EP 0 051 734 A1 offenbart ein Verfahren zur Herstellung von Aminotriazin-Alkoxylaten durch Reaktion von Aminotriazin-Hydroxiden mit Alkylenoxiden.

US 4,356,304 A beschreibt ein Verfahren zur Herstellung von oxyalkylierten Polyamino-1,3,5-Triazinen.

In WO 2009/144274 A2 geht es um amphiphile Moleküle mit einem Triazin-Kern.

Die Verfahren, die in den oben aufgeführten Dokumenten beschrieben wurden, weisen gravierende Nachteile auf: Entweder ist a) die Anwesenheit eines polaren und somit in der Regel auch hochsiedenden, inerten Lösungsmittels (Dimethylformamid oder Dimethylsulfoxid) erforderlich, das nach der Umsetzung aufwendig wieder vom Produkt abgetrennt werden muss, und/oder b) es ist die Anwesenheit mindestens eines hydroxy- oder aminogruppen-modifizierten Costarters erforderlich, was zu einer Mischung aus einem Aminotriazin-Alkoxilat und dem Costarter-Alkoxilat im Produkt führt. Das Aminotriazin-Alkoxilat ist somit mit dem Costarter-Alkoxilat verunreinigt. Da die Abtrennung der hochsiedenen polaren Lösungsmittel mit erheblichem verfahrenstechnischen Aufwand verbunden ist und die Anwesenheit eines Co-Starter-Alkoxilates sich negativ auf die Produkteigenschaften auswirkt, konnten sich derartige Produkte bislang nicht am Markt durchsetzen.

Somit war es Aufgabe der vorliegenden Erfindung, ein alternatives Verfahren zur Herstellung von Aminotriazin-Alkoxilaten zu entwickeln, das a) ohne die Verwendung von inerten Lösungsmitteln ablaufen kann und b) bei dem Aminotriazin-Alkoxilate erhältlich sind, die keine anderen Polyetherole als Verunreinigungen enthalten.

### Beschreibung der Erfindung:

Die oben genannte Aufgabe konnte überraschenderweise durch die Merkmalskombinationen der Ansprüche gelöst werden.

Der Gegenstand der vorliegenden Erfindung ist somit ein Verfahren zur Herstellung von Aminotriazin-Alkoxilaten, bei dem mindestens ein Aminotriazin-Alkoxilat (a) mit mindestens einem Aminotriazin (b) versetzt, wobei das Aminotriazin (b) 2,4,6-Triamino-1,3,5-triazin ist, und mit mindestens einem Alkylenoxid (c) zur Reaktion gebracht wird, wobei das Aminotriazin-Alkoxilat (a) herstellbar ist durch Reaktion mindestens eines Aminotriazins (d) mit mindestens einem Alkylenoxid (e) und/oder mindestens einem Alkylencarbonat (g), und optional mindestens einem Aminotriazin-Alkoxilat (f).

In einer bevorzugten Ausführungsform des erfindungsgemäßen Verfahrens zur Herstellung von Aminotriazin-Alkoxilaten ist das Aminotriazin-Alkoxilat (a) ausgewählt aus der Gruppe umfassend Reaktionsprodukte von mindestens einer Verbindung der folgenden Formel: 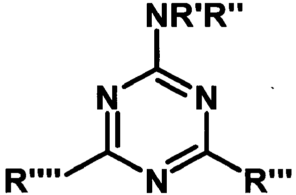 wobei R' und R" jeweils ausgewählt sind aus H, verzweigten oder linearen C1- bis C22-Verbindungen, Polypropylenglykol und Polyethylenglykol, R"' und R"" jeweils ausgewählt sind aus H, NR'R", OH, Halogenid, verzweigten oder linearen C1- bis C22-Ketten, C6R5, wobei R ausgewählt ist aus H, verzweigten oder linearen C1- bis C22-Alkylketten, Aryl, oder eine Mischung aus Verbindungen mit dieser Formel, mit mindestens einem Alkylenoxid (h).

Das Aminotriazin-Alkoxilat (a) des ersten Reaktionsansatzes kann nach einem der oben in den zitierten Patentschriften beschriebenen Verfahren hergestellt werden.

Darüber hinaus kann das Aminotriazin-Alkoxilat (a) des ersten Reaktionsansatzes durch folgende Verfahrensschritte hergestellt werden: 1.) Umsetzung eines Aminotriazins mit wässriger KOH- oder NaOH-Lösung mit anschließender Entfernung des Wassers durch Vakuumstripping, 2.) Reaktion des resultierenden Metallsalzes des Aminotriazins mit Alkylenoxiden, 3.) Neutralisation des Reaktionsproduktes (vorzugsweise mit Brönstedt-Säuren wie Phosphorsäure, Salzsäure, Schwefelsäure, aber auch CO₂ oder Makrosorb® (z. B. Makrosorb® MP5, ein Magnesiumsilikat der Firma INEOS Silicas, speziell entwickelt zur Aufreinigung von Polyolen)) , 4.) Entfernung der Neutralisationssalze aus Schritt 3 und des nicht abreagierten Aminotriazins aus Schritt 2 vom Aminotriazin-Alkoxilat durch Filtration. Das so neutralisierte Aminotriazin-Alkoxilat kann als Ausgangsprodukt (a) des oben beschriebenen Verfahrens eingesetzt werden.

Das Aminotriazin-Alkoxilat (a) kann in Anwesenheit oder in Abwesenheit eines Katalysators hergestellt werden.

In einer Ausführungsform des erfindungsgemäßen Verfahrens wird das Aminotriazin-Alkoxilat (a), das Ausgangsprodukt des erfindungsgemäßen Verfahrens, in Anwesenheit eines Lösungsmittels hergestellt. Bevorzugt werden in diesem Falle Reste des Lösungsmittels vor dem Einsatz des Aminotriazin-Alkoxilats (a) im erfindungsgemäßen Verfahren zur Herstellung von Aminotriazin-Alkoxilaten durch übliche Verfahren entfernt.

In einer weiteren Ausführungsform des erfindungsgemäßen Verfahrens wird das Aminotriazin-Alkoxilat (a) in Abwesenheit eines Lösungsmittels hergestellt.

Dem Fachmann sind ähnliche Verfahren auch unter der Bezeichnung "Heel-Verfahren" bekannt, die dadurch gekennzeichnet sind, dass im Folge-Reaktionsansatz ein Teil des Reaktionsproduktes aus dem vorherigen Reaktionsansatz zusammen mit der Ausgangskomponente, in diesem Falle das Aminotriazin, mit vorgelegt wird. Durch diesen Verfahrensschritt wird die Löslichkeit des Starters verbessert, da das Reaktionsprodukt als Lösungsvermittler fungiert, was dazu führt, dass die Reaktion vollständiger abläuft und kein unreagiertes Aminotriazin im Reaktionsprodukt zurückbleibt. Nicht abreagiertes Aminotriazin ist im Reaktionsprodukt von Nachteil, da es in fester Form im Reaktionsprodukt vorliegt und mit dem flüssigen Reaktionsprodukt phasensepariert.

Zudem ist in vielen Fällen beim erfindungsgemäßen Verfahren eine Zugabe eines Katalysators nicht erforderlich, da die Aminofunktionalitäten der Aminotriazine für den Anlagerungsschritt der Alkylenoxide autokatalytisch wirken.

Das erfindungsgemäße Verfahren bietet somit einen wirtschaftlichen Vorteil, u. a. da in vielen Fällen kein Katalysator erforderlich ist; außerdem können die Produkte in reiner Form und ohne zusätzlichen Reinigungsaufwand, der aufgrund hochsiedender Lösungsmittel oder Verunreinigungen durch Costarter bei konventionellen Verfahren nötig ist, direkt in der entsprechenden Anwendung eingesetzt werden. Im Gegensatz zu den bereits bekannten Heel-Verfahren kann in diesem Fall zudem auf den Zusatz eines Alkoxilierungskatalysators verzichtet werden.

Da die autokatalytische Umsetzung jedoch hinsichtlich des zu erreichenden Molekulargewichts in aller Regel beschränkt ist, ist die Weiterreaktion des Reaktionsproduktes aus dem beschriebenen Verfahren mit Alkylenoxid auch in Gegenwart von basischen Katalysatoren wie Alkalimetallhydroxiden, Alkalimetallalkoholaten oder Aminen, wie Dimethylethanolamin oder Imidazol, möglich.

Als Aminotriazine (d) kommen alle Aminotriazine in Frage, die mindestens eine, vorzugsweise mindestens zwei Aminogruppen im Molekül gebunden enthalten. Dies sind beispielsweise mit aliphatischen, cycloaliphatische oder aromatischen Resten mit 1 bis 18 Kohlenstoffatomen substituierte Aminotriazine, wie: 6-Methyl-, 6-Ethyl-, 6-n-Propyl-, 6-Isopropyl-, 6-Butyl-, 6-Hexyl-, 6-Nonyl-, 6-Stearyl-, 6-Butenyl-, 6-cyclohexyl-, 6-Phenyl-, 6-Dimethylamino-2,4-diamino-1,3,5-triazin. Ferner sind Aminotriazine mit Hydroxysubstituenten, wie z. B. das 6-Hydroxy-2,4-diamino-1,3,5-triazin (Ammelin) verwendbar. Vorzugsweise verwendet werden schwerlösliche und hochschmelzende Aminotriazine wie z. B. 6-Methyl-2,4-diamino-1,3,5-triazin, 6-Phenyl-2,4-diamino-1,3,5-triazin und insbesondere Melamin. Die Aminotriazine können einzeln oder in Form von Mischungen verwendet werden.

Als Aminotriazin (d) kann auch eine Verbindung der folgenden Formel: 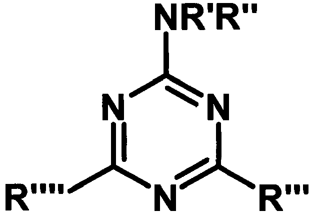 wobei R' und R" jeweils ausgewählt sind aus H, verzweigten oder linearen C1- bis C22-Verbindungen, Polypropylenglykol und Polyethylenglykol, R"' und R"" jeweils ausgewählt sind aus H, NR'R", OH, Halogenid, verzweigten oder linearen C1- bis C22-Ketten, C6R5, wobei R ausgewählt ist aus H, verzweigten oder linearen C1- bis C22-Alkylketten, Aryl, oder eine Mischung aus Verbindungen mit dieser Formel, eingesetzt werden.

Dabei können die Aminotriazine (b) und (d) gleich oder verschieden sein.
Bevorzugt kommt 2,4,6-Triamino-1,3,5-triazin (auch Melamin genannt) als Aminotriazin (d) zum Einsatz.

In einer bevorzugten Ausführungsform des erfindungsgemäßen Verfahrens zur Herstellung von Aminotriazin-Alkoxilaten ist das Aminotriazin-Alkoxilat (f) ausgewählt aus der Gruppe umfassend Reaktionsprodukte von mindestens einer Verbindung der folgenden Formel: 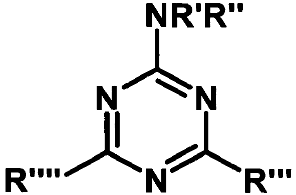 wobei R' und R" jeweils ausgewählt sind aus H, verzweigten oder linearen C1- bis C22-Verbindungen, Polypropylenglykol und Polyethylenglykol, R"' und R"" jeweils ausgewählt sind aus H, NR'R", OH, Halogenid, verzweigten oder linearen C1- bis C22-Ketten, C6R5, wobei R ausgewählt ist aus H, verzweigten oder linearen C1- bis C22-Alkylketten, Aryl, oder eine Mischung aus Verbindungen mit dieser Formel, mit mindestens einem Alkylenoxid (i).

In einer Ausführungsform des erfindungsgemäßen Verfahrens ist das Aminotriazin-Alkoxilat (f) vorhanden.
In einer anderen Ausführungsform des erfindungsgemäßen Verfahrens ist das Aminotriazin-Alkoxilat (f) nicht vorhanden.

Die Alkylenoxide (c), (e), (h) und (i) sind bevorzugt jeweils unabhängig voneinander ausgewählt aus der Gruppe umfassend Propylenoxid, Ethylenoxid, 1,2-Butylenoxid, 2,3-Butylenoxid, Isobutylenoxid, 1,2-Pentenoxid, Styroloxid, Epichlorhydrin, Glycidol und Mischungen daraus. Auch 2,3-Pentenoxid, 1,2-Hexenoxid, Cyclohexenoxid, Glycidylether, und/oder Butadienmonoxid oder Mischungen daraus können zum Einsatz kommen. Besonders bevorzugt sind dabei jeweils Propylenoxid und Ethylenoxid.

Das Akylencarbonat (g) ist bevorzugt ausgewählt aus der Gruppe umfassend Propylencarbonat, Ethylencarbonat, Glycerolcarbonat und Mischungen daraus.

Die Umsetzung der Aminotriazine mit Alkylenoxid in Gegenwart von Aminotriazin-Alkoxilaten erfolgt vorzugsweise bei Temperaturen zwischen 100 und 200 °C, besonders bevorzugt zwischen 150 und 180°C, in der Regel bei Drücken von 1 - 10 bar.

In einer bevorzugten Ausführungsform des erfindungsgemäßen Verfahrens erfolgt die Umsetzung von Aminotriazin-Alkoxilat (a) mit mindestens einem Aminotriazin (b) und mit mindestens einem Alkylenoxid (c) bei Temperaturen zwischen 100 und 200° C und bei Drücken von 1 - 10 bar in Abwesenheit eines Katalysators und in Abwesenheit eines Lösungsmittels.

Das Massenverhältnis zwischen Aminotriazin-Alkoxilat (a), das zusammen mit Aminotriazin im Reaktor vorgelegt wird, und dem Aminotriazin (b) beträgt (20% zu 80%) bis (95% zu 5%), vorzugsweise (40% zu 60%) bis (95% zu 5%).

Die Umsetzung der Aminotriazine mit Alkylenoxid in Gegenwart von Aminotriazin-Alkoxilaten kann in Anwesenheit von basischen Katalysatoren erfolgen. Als Katalysatoren können zum Beispiel Alkali- und Erdalkalimetallhydroxide bzw. -alkoholate eingesetzt werden. Darüber hinaus können Amine als Katalysatoren eingesetzt werden. Der Einsatz von Aminen hat insbesondere im Hinblick auf Polyurethananwendungen den verfahrenstechnischen Vorteil, dass der Katalysator aus dem Endprodukt nicht entfernt werden muss und somit kein zusätzlicher Neutralisation- und Filtrationsschritt anfällt. Als Amine werden bevorzugt tertiäre Amine eingesetzt; Beispiele für aminische Katalysatoren sind Trimethylamin (TMA), Tributylamin, Triethylamin (TEA), Dimethylethanolamin (DMEOA) und Dimethylcyclohexylamin (DMCHA), Imidazol und substituierte Imidazolderivate, vorzugsweise Dimethylethanolamin.

Besonders bevorzugt sind DMEOA (Dimethylethanolamin), Imidazol.

Auch Carbene, bevorzugt N-heterocyclische Carbene, können als Katalysatoren eingesetzt werden.

In einer weiteren bevorzugten Ausführungsform erfolgt die Umsetzung der Aminotriazine mit Alkylenoxid ohne die Anwesenheit eines Katalysators. Da die Aminotriazine selber katalytisch wirksame funktionelle Gruppen tragen, kann die Reaktion somit autokatalytisch erfolgen. Auch in diesem Fall hat man den verfahrenstechnischen Vorteil, dass keine Neutralisation und Filtration notwendig ist.

Das Reaktionsprodukt wird in der Regel nach beendeter Reaktion von Restmonomer und anderen flüchtigen Komponenten durch Vakuumstripping befreit. Das Stripping kann zum Beispiel mittels eines inerten Gases (z.B. Stickstoff) oder auch mit Wasserdampf erfolgen. Wenn Metallhydroxide oder Metallalkoholate als Katalysatoren bei der Umsetzung mit Alkylenoxiden verwendet wurden, werden diese zum Beispiel durch Zugabe von Brönstedtsäuren neutralisiert und die Metallsalze durch Filtration vom Reaktionsprodukt abgetrennt.

Die Anlagerung der Alkylenoxide kann blockweise oder statistisch erfolgen. Es können sowohl reine Alkylenoxide als auch Alkylenoxid-Gemische verwendet werden.

Die Umsetzung des Aminotriazin-Alkoxilats (a) mit mindestens einem Aminotriazin (b) und mit mindestens einem Alkylenoxid (c) zur Herstellung von Aminotriazin-Alkoxilaten erfolgt erfindungsgemäß bevorzugt ohne Verwendung eines inerten Lösungsmittels.

Die Herstellung des Aminotriazin-Alkoxilats (a) kann jedoch - wie bereits oben erwähnt - optional in Anwesenheit eines inerten Lösungsmittels erfolgen.

Erfindungsgemäß werden im Verfahren zur Herstellung von Aminotriazin-Alkoxilaten durch Umsetzung mindestens eines Aminotriazin-Alkoxilats (a) mit mindestens einem Aminotriazin (b) und mit mindestens einem Alkylenoxid (c) bevorzugt keine Costarter, wie z. B. reaktive Lösungsmittel mit Hydroxy- oder Aminogruppen, verwendet.

Die Herstellung des Aminotriazin-Alkoxilats (a) kann jedoch optional in Anwesenheit eines hydroxy- und/oder aminofunktionellen und somit reaktiven Costarters erfolgen.

Das Verfahren kann diskontinuierlich im Batch-Verfahren oder auch im Semibatch-Verfahren durchgeführt werden. Weiterhin kann das Verfahren auch kontinuierlich durchgeführt werden, indem das Aminotriazin oder eine Mischung aus dem Aminotriazin-Alkoxilat mit dem Aminotriazin kontinuierlich dem Reaktionskessel zugeführt werden und das Produkt kontinuierlich entnommen wird.

Die mit dem beschriebenen Verfahren erhältlichen Aminotriazin-Alkoxilate haben in der Regel einen Hydroxywert von 10 - 600 mg KOH/ g, vorzugsweise zwischen 50 - 500 mg KOH/ g. Die Funktionalitäten der erfindungsgemäßen Aminotriazine liegen in der Reget zwischen 2-6, vorzugsweise zwischen 3-6. Die Molekulargewichte liegen in der Regel zwischen 300 und 15.000 g/mol, vorzugsweise zwischen 400 und 5000 g/mol.

Die erfindungsgemäß erhaltenen Aminotriazin-Alkoxilate können für verschiedenste Zwecke verwendet werden, zum Beispiel für:
1.) Formaldehyd-freie Ledergerbstoffe und/ oder Ledernachgerbstoffe
2.) Oberflächenaktive Materialien (Tenside, Emulgatoren)
3.) Dispergiermittel für wässrige Systeme, für organische Lösungsmittel oder für Polyetherpolyole
4.) Solubilisatoren für wasserunlösliche Verbindungen (Agro- oder Pharma-Adjuvanz)
5.) Polyetherol-Komponente in Polyurethansystem-Formulierungen, vorzugsweise in Polyurethan-Hartschaum und Polyurethan-Coatings-Formulierungen, besonders bevorzugt in Polyurethan-Hartschaum-Systemen

Zudem können die nach dem erfindungsgemäßen Verfahren erhältlichen Aminotriazin-Alkoxilate als Vorlage in einem Verfahren zur Herstellung weiterer Aminotriazin-Alkoxilate eingesetzt werden. Insbesondere können die nach dem erfindungsgemäßen Verfahren erhältlichen Aminotriazin-Alkoxilate auch als Vorlage in einem Verfahren, das dem erfindungsgemäßen Verfahren analog ist, zur Herstellung weiterer Aminotriazin-Alkoxilate eingesetzt werden. In diesem Falle dienen die erfindungsgemäß herstellbaren Aminotriazin-Alkoxilate wiederum als Aminotriazin-Alkoxilat (a) gemäß Anspruch 1.

Die erfindungsgemäß herstellbaren Aminotriazin-Alkoxilate eignen sich, wie erwähnt, besonders gut zur Bildung oberflächenaktiver Mittel vom Aktiv-Typ. Sie sind daher gut geeignet zum Befeuchten oder Weichmachen von Wolle, Baumwolle oder Celluloseacetat, Cellulosenitrat, Viskose und ähnlichen Materialien. Auch zur Emulsifizierung von Mineralölen, Glyceriden, Fetten, Ölen und Ähnlichem sind sie geeignet. Die erfindungsgemäß herstellbaren Aminotriazin-Alkoxilate finden Anwendung bei der Formulierung von Drucktinten, Färbepasten, Trockenbädern, Lederzubereitungen und Flotationsmitteln. Durch Anwendung der erfindungsgemäß herstellbaren Aminotriazin-Alkoxilate ist es möglich, Fülle, Widerstandsfähigkeit gegenüber Auflösung und erhöhte Nassfestigkeit bei Rayon und anderen Fasern sicher zu stellen. Die erfindungsgemäß herstellbaren Aminotriazin-Alkoxilate reduzieren auch die Neigung behandelter Stoffmaterialien, sich zu falten und zu knittern.

### Beispiele

Es werden nun einige Beispiele zur Illustration der Erfindung dargestellt. Diese sind nur exemplarisch zu verstehen und sollen den Umfang der vorliegenden Erfindung nicht einschränken.

### Beispiel 1: Herstellung eines Aminotriazin-Ethoxilats (a1)

46.2 g Melamin wurden in einem 300mL-Stahlautoklaven mit 1,44 g wässriger KOH-Lösung (50%ig) versetzt und die Reaktionsmischung dreimal mit Stickstoff inertisiert. Anschließend wurde die Reaktionsmischung durch Evakuierung bei 110°C auf 10 mbar vom Wasser befreit. Danach erfolgte die Dosierung von 193,8 g Ethylenoxid innerhalb von 4 Stunden. Nachdem die Ethylenoxid-Abschlagsmenge erreicht wurde, folgte eine Nachreaktion, bis der Druck konstant verlief. Nach der Abreaktion wurde das Restmonomer bei 110°C für 30 min bei 10 mbar abdestilliert und das Rohprodukt bei Raumtemperatur abgelassen. Das Rohprodukt enthielt noch unreagiertes Melamin in fester Form, das zunächst durch Filtration vom flüssigen Melamin-Ethoxilat abgetrennt wurde. Das basische Filtrat wurde sodann mit 5% Wasser und 3% Makrosorb^{®} versetzt, für 60 min bei 90 °C erwärmt, bei 3 mbar und 120°C vom Wasser und anderen flüchtigen Komponenten befreit und schließlich mit Hilfe eines Druckfilters filtriert. Das neutralisierte Melamin-Ethoxilat hatte eine Hydroxyzahl von 174,0 mg KOH/g und eine Restalkalität von < 10 ppm K⁺ (Bestimmung per Atom-Absorptions-Spektrometrie, AAS) und wurde direkt als Aminotriazin-Alkoxilat (a1) für das erfindungsgemäße Verfahren nach den unten beschriebenen Versuchsvorschriften verwendet.

### Beispiel 2: Herstellung eines Melamin-Ethoxilates unter Verwendung des Produktes aus Beispiel 1 als Vorlage

63 g des Melamin-Ethoxilates (a1) aus Beispiel 1 wurden zusammen mit 7 g Melamin in einen 300mL-Autoklaven vorgelegt und die Mischung dreimal mit Stickstoff inertisiert. Anschließend wurde der Reaktionsansatz auf 110°C erwärmt und 104 g Ethylenoxid innerhalb von 7,5 Stunden zudosiert. Nach beendeter Dosierung erfolgte eine Abreaktion von 3 Stunden. Nach beendeter Abreaktion wurde restliches Monomer im Vakuum entfernt und das Produkt bei Raumtemperatur abgelassen. Es wurden 170 g einer homogenen Flüssigkeit erhalten, die eine Hydroxyzahl von 142 mg KOH/g aufwies.

### Beispiel 3: Herstellung eines Melamin-Ethoxilates unter Verwendung des Produktes aus Beispiel 2 als Vorlage

63 g des Melamin-Ethoxilates aus Beispiel 2 wurden zusammen mit 7 g Melamin in einen 300mL-Autoklaven vorgelegt und die Mischung dreimal mit Stickstoff inertisiert. Anschließend wurde der Reaktionsansatz auf 160°C erwärmt und 104 g Ethylenoxid innerhalb von 4 Stunden zudosiert. Nach beendeter Dosierung erfolgte eine Abreaktion von 3 Stunden. Nach beendeter Abreaktion wurde restliches Monomer im Vakuum entfernt und das Produkt bei Raumtemperatur abgelassen. Es wurden 170 g einer homogenen Flüssigkeit erhalten, die eine Hydroxyzahl von 147 mg KOH/g aufwies.

### Beispiel 4: Herstellung eines Melamin-Ethoxilates unter Verwendung des Produktes aus Beispiel 3 als Vorlage

40 g des Melamin-Ethoxilates aus Beispiel 3 wurden zusammen mit 10 g Melamin in einen 300mL-Autoklaven vorgelegt und die Mischung dreimal mit Stickstoff inertisiert. Anschließend wurde der Reaktionsansatz auf 160°C erwärmt und 171,6 g Ethylenoxid innerhalb von 4 Stunden zudosiert. Nach beendeter Dosierung erfolgte eine Abreaktion von 3 Stunden. Nach beendeter Abreaktion wurde restliches Monomer im Vakuum entfernt und das Produkt bei Raumtemperatur abgelassen. Es wurden 210 g einer homogenen Flüssigkeit erhalten, die eine Hydroxyzahl von 154,6 mg KOH/g aufwies.

### Beispiel 5: Herstellung eines Melamin-Ethoxilates unter Verwendung des Produktes aus Beispiel 4 als Vorlage

28 g des Melamin-Ethoxilates aus Beispiel 4 wurden zusammen mit 12 g Melamin in einen 300mL-Autoklaven vorgelegt und die Mischung dreimal mit Stickstoff inertisiert. Anschließend wurde der Reaktionsansatz auf 160°C erwärmt und 196 g Ethylenoxid innerhalb von 7,5 Stunden zudosiert. Nach beendeter Dosierung erfolgte eine Abreaktion von 3 Stunden. Nach beendeter Abreaktion wurde restliches Monomer im Vakuum entfernt und das Produkt bei Raumtemperatur abgelassen. Es wurden 230 g einer homogenen Flüssigkeit erhalten, die eine Hydroxyzahl von 161,3 mg KOH/g aufwies.

### Beispiel 6: Herstellung eines Melamin-Ethoxilates unter Verwendung des Produktes aus Beispiel 5 als Vorlage

20,3 g des Melamin-Ethoxilates aus Beispiel 5 wurden zusammen mit 13,5 g Melamin in einen 300mL-Autoklaven vorgelegt und die Mischung dreimal mit Stickstoff inertisiert. Anschließend wurde der Reaktionsansatz auf 160°C erwärmt und 206,2 g Ethylenoxid innerhalb von 10 Stunden zudosiert. Nach beendeter Dosierung erfolgte eine Abreaktion von 3 Stunden. Nach beendeter Abreaktion wurde restliches Monomer im Vakuum entfernt und das Produkt bei Raumtemperatur abgelassen. Es wurden 230 g einer homogenen Flüssigkeit erhalten, die eine Hydroxyzahl von 171,2 mg KOH/g aufwies.

### Beispiel 7: Herstellung eines Melamin-Ethoxilates unter Verwendung des Produktes aus Beispiel 6 als Vorlage

14,6 g des Melamin-Ethoxilates aus Beispiel 6 wurden zusammen mit 14,5 g Melamin in einen 300mL-Autoklaven vorgelegt und die Mischung dreimal mit Stickstoff inertisiert. Anschließend wurde der Reaktionsansatz auf 160°C erwärmt und 209 g Ethylenoxid innerhalb von 12 Stunden zudosiert. Nach beendeter Dosierung erfolgte eine Abreaktion von 3 Stunden. Nach beendeter Abreaktion wurde restliches Monomer im Vakuum entfernt und das Produkt bei Raumtemperatur abgelassen. Es wurden 228 g einer homogenen Flüssigkeit erhalten, die eine Hydroxyzahl von 162,5 mg KOH/g aufwies.

### Beispiel 8: Herstellung eines Melamin-Ethoxilates unter Verwendung des Produktes aus Beispiel 7 als Vorlage

14,6 g des Melamin-Ethoxilates aus Beispiel 7 wurden zusammen mit 14,5 g Melamin in einen 300mL-Autoklaven vorgelegt und die Mischung dreimal mit Stickstoff inertisiert. Anschließend wurde der Reaktionsansatz auf 160°C erwärmt und 209 g Ethylenoxid innerhalb von 7,5 Stunden zudosiert. Nach beendeter Dosierung erfolgte eine Abreaktion von 3 Stunden. Nach beendeter Abreaktion wurde restliches Monomer im Vakuum entfernt und das Produkt bei Raumtemperatur abgelassen. Es wurden 228 g einer homogenen Flüssigkeit erhalten, die eine Hydroxyzahl von 162,5 mg KOH/g aufwies.

### Beispiel 9: Herstellung eines Aminotriazin-Propoxilates (a2)

20.0 g Melamin wurden in einem 300mL-Stahlautoklaven mit 2,4 g wässriger KOH-Lösung (50%ig) versetzt und die Reaktionsmischung dreimal mit Stickstoff inertisiert. Anschließend wurde die Reaktionsmischung durch Evakuierung bei 110°C auf 10 mbar vom Wasser befreit. Danach erfolgte die Dosierung von 220,0 g Propylenoxid innerhalb von 20 Stunden. Nachdem die Propylenoxid-Abschlagsmenge erreicht wurde, folgte eine Nachreaktion bis der Druck konstant verlief. Nach der Abreaktion wurde das Restmonomer bei 110°C für 30 min bei 10 mbar abdestilliert und das Rohprodukt bei Raumtemperatur abgelassen. Das Rohprodukt enthielt noch unreagiertes Melamin in fester Form, das zunächst durch Filtration vom flüssigen Melamin-Propoxilat abgetrennt wurde. Das basische Filtrat wurde sodann mit 5% Wasser und 3% Makrosorb^{®} versetzt, für 60 min bei 90 °C erwärmt, bei 3 mbar und 120°C vom Wasser und anderen flüchtigen Komponenten befreit und schließlich mit Hilfe eines Druckfilters filtriert. Das neutralisierte Melamin-Propoxilat hatte eine Hydroxyzahl von 238,4 mg KOH/g und eine Restalkalität von < 10 ppm K⁺ (Bestimmung per Atom-Absorptions-Spektrometrie, AAS) und wurde direkt als Einsatzstoff für unten beschriebene Folgeansätze verwendet.

### Beispiel 10: Herstellung eines Melamin-Propoxilates unter Verwendung des Produktes aus Beispiel 9 als Vorlage

106,1 g des Melamin-Propoxilates (a2) aus Beispiel 9 wurden zusammen mit 11,8 g Melamin in einen 300mL-Autoklaven vorgelegt und die Mischung dreimal mit Stickstoff inertisiert. Anschließend wurde der Reaktionsansatz auf 160°C erwärmt und 122,2 g Propylenoxid innerhalb von 9 Stunden zudosiert. Nach beendeter Dosierung erfolgte eine Abreaktion von 10 Stunden. Nach beendeter Abreaktion wurde restliches Monomer im Vakuum entfernt und das Produkt bei Raumtemperatur abgelassen. Es wurden 230 g einer homogenen Flüssigkeit erhalten, die eine Hydroxyzahl von 273 mg KOH/g aufwies.

### Beispiel 11: Herstellung eines Melamin-Propoxilates unter Verwendung des Produktes aus Beispiel 10 als Vorlage

63,2 g des Melamin-Propoxilates aus Beispiel 10 wurden zusammen mit 15,8 g Melamin in einen 300mL-Autoklaven vorgelegt und die Mischung dreimal mit Stickstoff inertisiert. Anschließend wurde der Reaktionsansatz auf 160°C erwärmt und 161 g Propylenoxid innerhalb von 12 Stunden zudosiert. Nach beendeter Dosierung erfolgte eine Abreaktion von 3 Stunden. Nach beendeter Abreaktion wurde restliches Monomer im Vakuum entfernt und das Produkt bei Raumtemperatur abgelassen. Es wurden 230 g einer homogenen Flüssigkeit erhalten, die eine Hydroxyzahl von 267 mg KOH/g aufwies.

### Beispiel 12: Herstellung eines Melamin-Propoxilates unter Verwendung des Produktes aus Beispiel 11 als Vorlage

63,2 g des Melamin-Propoxilates aus Beispiel 10 wurden zusammen mit 15,8 g Melamin in einen 300mL-Autoklaven vorgelegt und die Mischung dreimal mit Stickstoff inertisiert. Anschließend wurde der Reaktionsansatz auf 160°C erwärmt und 161 g Propylenoxid innerhalb von 11 Stunden zudosiert. Nach beendeter Dosierung erfolgte eine Abreaktion von 3 Stunden. Nach beendeter Abreaktion wurde restliches Monomer im Vakuum entfernt und das Produkt bei Raumtemperatur abgelassen. Es wurden 230 g einer homogenen Flüssigkeit erhalten, die eine Hydroxyzahl von 270 mg KOH/g aufwies.

### Beispiel 13: Herstellung eines Melamin-Propoxilates unter Verwendung des Produktes aus Beispiel 12 als Vorlage

63,2 g des Melamin-Propoxilates aus Beispiel 12 wurden zusammen mit 15,8 g Melamin in einen 300 mL-Autoklaven vorgelegt und die Mischung dreimal mit Stickstoff inertisiert. Anschließend wurde der Reaktionsansatz auf 160°C erwärmt und 140 g Propylenoxid innerhalb von 8 Stunden zudosiert. Nach beendeter Dosierung erfolgte eine Abreaktion von 3 Stunden. Nach beendeter Abreaktion wurde restliches Monomer im Vakuum entfernt und das Produkt bei Raumtemperatur abgelassen. Es wurden 210 g einer homogenen Flüssigkeit erhalten, die eine Hydroxyzahl von 285 mg KOH/g aufwies.

### Beispiel 14: Herstellung eines Melamin-Propoxilates unter Verwendung eines Melamin-Ethoxilates als Vorlage, das hergestellt wurde durch Umsetzung von Melamin mit Ethylencarbonat

35,2 g eines Melamin-Ethoxilates (a3) mit einer Hydroxyzahl von 515,8 mg KOH/g, das durch Umsetzung von Melamin mit Ethylencarbonat analog der Patentschrift US 3328321 hergestellt wurde, wurden zusammen mit 3,5 g Melamin in einen 300mL-Autoklaven vorgelegt und die Mischung dreimal mit Stickstoff inertisiert. Anschließend wurde der Reaktionsansatz auf 100°C erwärmt und 100 g Propylenoxid innerhalb von 2 Stunden zudosiert. Nach beendeter Dosierung erfolgte eine Abreaktion von 3 Stunden. Nach beendeter Abreaktion wurde restliches Monomer im Vakuum entfernt und das Produkt bei Raumtemperatur abgelassen. Es wurden 128 g einer homogenen Flüssigkeit erhalten, die eine Hydroxyzahl von 195 mg KOH/g aufwies.

Die Versuchsergebnisse zeigen somit, dass mit dem erfindungsgemäßen Verfahren homogene Aminotriazin-Alkoxilate zugänglich sind, die ohne Verwendung von nicht Aminotriazin-haltigen Costartern und ohne Verwendung eines inerten Lösungsmittels zugänglich sind. Somit handelt es sich bei den Produkten um reine Aminotriazin-Alkoxilate, die durch ein einfaches und kostengünstiges Verfahren herstellbar sind.

## Patentansprüche

1. Verfahren zur Herstellung von Aminotriazin-Alkoxilaten, bei dem mindestens ein Aminotriazin-Alkoxilat (a) mit mindestens einem Aminotriazin (b) versetzt, wobei das Aminotriazin (b) 2,4,6-Triamino-1,3,5-triazin ist, und mit mindestens einem Alkylenoxid (c) zur Reaktion gebracht wird, wobei das Aminotriazin-Alkoxilat (a) herstellbar ist durch Reaktion mindestens eines Amienotriazins (d) mit mindestens einem Alkylenoxid (e) und/oder mindestens einem Alkylencarbonat (g), und optional mindestens einem Aminotriazin-Alkoxilat (f).

2. Verfahren zur Herstellung von Aminotriazin-Alkoxilaten nach Anspruch 1, wobei das Aminotriazin-Alkoxilat (a) ausgewählt ist aus der Gruppe umfassend Reaktionsprodukte von mindestens einer Verbindung der folgenden Formel: 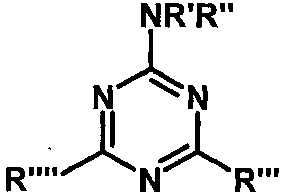
wobei R' und R" jeweils ausgewählt sind aus H, verzweigten oder linearen C1- bis C22-Verbindungen, Polypropylenglykol und Polyethylenglykol, R"' und R"" jeweils ausgewählt sind aus H, NR'R", OH, Halogenid, verzweigten oder linearen C1- bis C22-Ketten, C6R5,
wobei R ausgewählt ist aus H, verzweigten oder linearen C1- bis C22-Alkylketten, Aryl, oder eine Mischung aus Verbindungen mit dieser Formel, mit mindestens einem Alkylenoxid (h).

3. Verfahren zur Herstellung von Aminotriazin-Alkoxilaten nach Anspruch 1 oder 2, wobei das Aminotriazin (d) ausgewählt ist aus Verbindungen der folgenden Formel: 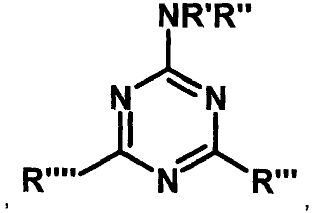
wobei R' und R" jeweils ausgewählt sind aus H, verzweigten oder linearen C1- bis C22-Verbindungen, Polypropylenglykol und Polyethylenglykol, R"' und R"" jeweils ausgewählt sind aus H, NR'R", OH, Halogenid, verzweigten oder linearen C1- bis C22-Ketten, C6R5,
wobei R ausgewählt ist aus H, verzweigten oder linearen C1- bis C22-Alkylketten, Aryl, oder eine Mischung aus Verbindungen mit dieser Formel.

4. Verfahren zur Herstellung von Aminotriazin-Alkoxilaten nach einem der vorangehenden Ansprüche, wobei das Aminotriazin (d) 2,4,6-Triamino-1,3,5-triazin ist.

5. Verfahren zur Herstellung von Aminotriazin-Alkoxilaten nach einem der vorangehenden Ansprüche, wobei das Massenverhältnis zwischen Aminotriazin-Alkoxilat (a) und dem Aminotriazin (b) 20:80 bis 95:5, vorzugsweise 40:60 bis 95:5 beträgt.

6. Verfahren zur Herstellung von Aminotriazin-Alkoxilaten nach einem der vorangehenden Ansprüche, wobei das Aminotriazin-Alkoxilat (f) ausgewählt ist aus der Gruppe umfassend Reaktionsprodukte von mindestens einer Verbindung der folgenden Formel: 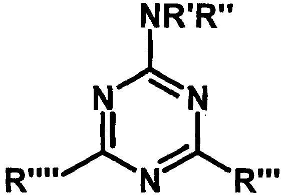
wobei R' und R" jeweils ausgewählt sind aus H, verzweigten oder linearen C1- bis C22-Verbindungen, Polypropylenglykol und Polyethylenglykol, R"' und R"" jeweils ausgewählt sind aus H, NR'R", OH, Halogenid, verzweigten oder linearen C1- bis C22-Ketten, C6R5,
wobei R ausgewählt ist aus H, verzweigten oder linearen C1- bis C22-Alkylketten, Aryl, oder eine Mischung aus Verbindungen mit dieser Formel, mit mindestens einem Alkylenoxid (i).

7. Verfahren zur Herstellung von Aminotriazin-Alkoxilaten nach einem der vorangehenden Ansprüche, wobei das Aminotriazin-Alkoxilat (f) vorhanden ist.

8. Verfahren zur Herstellung von Aminotriazin-Alkoxilaten nach einem der Ansprüche 1 bis 6, wobei das Aminotriazin-Alkoxilat (f) nicht vorhanden ist.

9. Verfahren zur Herstellung von Aminotriazin-Alkoxilaten nach einem der vorangehenden Ansprüche, wobei die Alkylenoxide (c), (e), (h) und (i) jeweils unabhängig voneinander ausgewählt sind aus der Gruppe umfassend Propylenoxid, Ethylenoxid, 1,2-Butylenoxid, 2,3-Butylenoxid, Isobutylenoxid, 1,2-Pentenoxid, Styroloxid, Epichlorhydrin, Glycidol und Mischungen daraus, bevorzugt Propylenoxid oder Ethylenoxid.

10. Verfahren zur Herstellung von Aminotriazin-Alkoxilaten nach einem der vorangehenden Ansprüche, wobei das Akylencarbonat (g) ausgewählt ist aus der Gruppe umfassend Propylencarbonat, Ethylencarbonat, Glycerolcarbonat und Mischungen daraus.

11. Verfahren zur Herstellung von Aminotriazin-Alkoxilaten nach einem der vorangehenden Ansprüche, wobei das Aminotriazin-Alkoxilat (a) in Anwesenheit eines Lösungsmittels hergestellt wird.

12. Verfahren zur Herstellung von Aminotriazin-Alkoxilaten nach einem der Ansprüche 1 bis 10, wobei das Aminotriazin-Alkoxilat (a) in Abwesenheit eines Lösungsmittels hergestellt wird.

13. Verfahren zur Herstellung von Aminotriazin-Alkoxilaten nach einem der vorangehenden Ansprüche, wobei die Umsetzung von Aminotriazin-Alkoxilat (a) mit mindestens einem Aminotriazin (b) und mit mindestens einem Alkylenoxid (c) bei Temperaturen zwischen 100 und 200° C und bei Drücken von 1-10 bar in Abwesenheit eines Katalysators und in Abwesenheit eines Lösungsmittels durchgeführt wird.

## Claims

1. A process for preparing aminotriazine alkoxylates, which comprises at least one aminotriazine alkoxylate (a) being admixed with at least one aminotriazine (b), wherein the aminotriazine (b) is 2,4,6-triamino-1,3,5-triazine, and reacted with at least one alkylene oxide (c), wherein the aminotriazine alkoxylate (a) is obtainable by reacting at least one aminotriazine (d) with at least one alkylene oxide (e) and/or at least one alkylene carbonate (g), and optionally at least one aminotriazine alkoxylate (f).

2. The process for preparing aminotriazine alkoxylates according to claim 1 wherein the aminotriazine alkoxylate (a) is selected from the group comprising reaction products of at least one compound of the following formula: 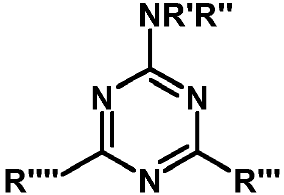 where R' and R" are each selected from H, branched or linear C1-C22 compounds, polypropylene glycol and polyethylene glycol, R"' and R"" are each selected from H, NR'R", OH, halide, branched or linear C1-C22 chains, C6R5, where R is selected from H, branched or linear C1-C22 alkyl chains, aryl, or a mixture of compounds having this formula, with at least one alkylene oxide (h).

3. The process for preparing aminotriazine alkoxylates according to claim 1 or 2 wherein the aminotriazine (d) is selected from compounds of the following formula: 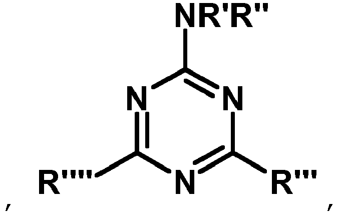 where R' and R" are each selected from H, branched or linear C1-C22 compounds, polypropylene glycol and polyethylene glycol, R"' and R"" are each selected from H, NR'R", OH, halide, branched or linear C1-C22 chains, C6R5, where R is selected from H, branched or linear C1-C22 alkyl chains, aryl, or a mixture of compounds having this formula.

4. The process for preparing aminotriazine alkoxylates according to any preceding claim wherein the aminotriazine (d) is 2,4,6-triamino-1,3,5-triazine.

5. The process for preparing aminotriazine alkoxylates according to any preceding claim wherein the mass ratio between aminotriazine alkoxylate (a) and the aminotriazine (b) is in the range from 20:80 to 95:5 and preferably in the range from 40:60 to 95:5.

6. The process for preparing aminotriazine alkoxylates according to any preceding claim wherein the aminotriazine alkoxylate (f) is selected from the group comprising reaction products of at least one compound of the following formula: 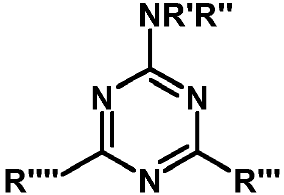 where R' and R" are each selected from H, branched or linear C1-C22 compounds, polypropylene glycol and polyethylene glycol, R"' and R"" are each selected from H, NR'R", OH, halide, branched or linear C1-C22 chains, C6R5, where R is selected from H, branched or linear C1-C22 alkyl chains, aryl, or a mixture of compounds having this formula, with at least one alkylene oxide (i).

7. The process for preparing aminotriazine alkoxylates according to any preceding claim wherein the aminotriazine alkoxylate (f) is present.

8. The process for preparing aminotriazine alkoxylates according to any one of claims 1 to 6 wherein the aminotriazine alkoxylate (f) is not present.

9. The process for preparing aminotriazine alkoxylates according to any preceding claim wherein the alkylene oxides (c), (e), (h) and (i) are each independently selected from the group comprising propylene oxide, ethylene oxide, 1,2-butylene oxide, 2,3-butylene oxide, isobutylene oxide, 1,2-pentene oxide, styrene oxide, epichlorohydrin, glycidol and mixtures thereof, preferably propylene oxide or ethylene oxide.

10. The process for preparing aminotriazine alkoxylates according to any preceding claim wherein the alkylene carbonate (g) is selected from the group comprising propylene carbonate, ethylene carbonate, glycerol carbonate and mixtures thereof.

11. The process for preparing aminotriazine alkoxylates according to any preceding claim wherein the aminotriazine alkoxylate (a) is obtained in the presence of a solvent.

12. The process for preparing aminotriazine alkoxylates according to any of claims 1 to 10 wherein the aminotriazine alkoxylate (a) is obtained in the absence of a solvent.

13. The process for preparing aminotriazine alkoxylates according to any preceding claim wherein the reaction of aminotriazine alkoxylate (a) with at least one aminotriazine (b) and with at least one alkylene oxide (c) is carried out at temperatures between 100 and 200°C and at pressures of 1 - 10 bar in the absence of a catalyst and in the absence of a solvent.

## Revendications

1. Procédé pour la préparation d'alcoxylates d'aminotriazine, dans lequel on additionne au moins un alcoxylate d'aminotriazine (a) d'au moins une aminotriazine (b), l'aminotriazine (b) étant la 2,4,6-triamino-1,3,5-triazine et on fait réagir avec au moins un oxyde d'alkylène (c), l'alcoxylate d'aminotriazine (a) pouvant être préparé par réaction d'au moins une aminotriazine (d) avec au moins un oxyde d'alkylène (e) et/ou au moins un carbonate d'alkylène (g) et éventuellement au moins un alcoxylate d'aminotriazine (f).

2. Procédé pour la préparation d'alcoxylates d'aminotriazine selon la revendication 1, l'alcoxylate d'aminotriazine (a) étant choisi dans le groupe comprenant les produits de réaction d'au moins un composé de formule suivante : 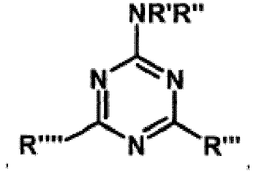 dans laquelle R' et R" sont à chaque choisis parmi H, des composés ramifiés ou linéaires en C₁-C₂₂, le polypropylèneglycol et le polyéthylèneglycol, R"' et R"" sont à chaque fois choisis parmi H, NR'R", OH, halogénure, des chaînes ramifiées ou linéaires en C₁-C₂₂, C₆R₅, R étant choisi parmi H, des chaînes alkyle ramifiées ou linéaires en C₁-C₂₂, aryle, ou d'un mélange de composés de cette formule avec au moins un oxyde d'alkylène (h).

3. Procédé pour la préparation d'alcoxylates d'aminotriazine selon la revendication 1 ou 2, l'aminotriazine (d) étant choisie dans le groupe des composés de formule suivante : 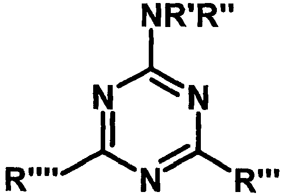 dans laquelle R' et R" sont à chaque choisis parmi H, des composés ramifiés ou linéaires en C₁-C₂₂, le polypropylèneglycol et le polyéthylèneglycol, R"' et R"" sont à chaque fois choisis parmi H, NR'R", OH, halogénure, des chaînes ramifiées ou linéaires en C₁-C₂₂, C₆R₅, R étant choisi parmi H, des chaînes alkyle ramifiées ou linéaires en C₁-C₂₂, aryle ou un mélange de composés de cette formule.

4. Procédé pour la préparation d'alcoxylates d'aminotriazine selon l'une quelconque des revendications précédentes, l'aminotriazine (d) étant la 2,4,6-triamino-1,3,5-triazine.

5. Procédé pour la préparation d'alcoxylates d'aminotriazine selon l'une quelconque des revendications précédentes, le rapport massique entre l'alcoxylate d'aminotriazine (a) et l'aminotriazine (b) étant de 20:80 à 95:5, de préférence de 40:60 à 95:5.

6. Procédé pour la préparation d'alcoxylates d'aminotriazine selon l'une quelconque des revendications précédentes, l'alcoxylate d'aminotriazine (f) étant choisi dans le groupe comprenant les produits de réaction d'au moins un composé de formule suivante : 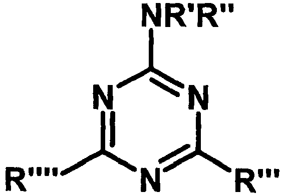 dans laquelle R' et R" sont à chaque choisis parmi H, des composés ramifiés ou linéaires en C₁-C₂₂, le polypropylèneglycol et le polyéthylèneglycol, R"' et R"" sont à chaque fois choisis parmi H, NR'R", OH, halogénure, des chaînes ramifiées ou linéaires en C₁-C₂₂, C₆R₅, R étant choisi parmi H, des chaînes alkyle ramifiées ou linéaires en C₁-C₂₂, aryle, ou d'un mélange de composés de cette formule avec au moins un oxyde d' alkylène (i).

7. Procédé pour la préparation d'alcoxylates d'aminotriazine selon l'une quelconque des revendications précédentes, l'alcoxylate d'aminotriazine (f) étant présent.

8. Procédé pour la préparation d'alcoxylates d'aminotriazine selon l'une quelconque des revendications 1 à 6, l'alcoxylate d'aminotriazine (f) n'étant pas présent.

9. Procédé pour la préparation d'alcoxylates d'aminotriazine selon l'une quelconque des revendications précédentes, les oxydes d'alkylène (c), (e), (h) et (i) étant choisis, à chaque fois indépendamment les uns des autres, dans le groupe comprenant l'oxyde de propylène, l'oxyde d'éthylène, l'oxyde de 1,2-butylène, l'oxyde de 2,3-butylène, l'oxyde d'isobutylène, l'oxyde de 1,2-pentène, l'oxyde de styrène, l'épichlorhydrine, le glycidol et leurs mélanges, de préférence l'oxyde de propylène ou l'oxyde d'éthylène.

10. Procédé pour la préparation d'alcoxylates d'aminotriazine selon l'une quelconque des revendications précédentes, le carbonate d'alkylène (g) étant choisi dans le groupe comprenant le carbonate de propylène, le carbonate d'éthylène, le carbonate de glycérol et leurs mélanges.

11. Procédé pour la préparation d'alcoxylates d'aminotriazine selon l'une quelconque des revendications précédentes, l'alcoxylate d'aminotriazine (a) étant préparé en présence d'un solvant.

12. Procédé pour la préparation d'alcoxylates d'aminotriazine selon l'une quelconque des revendications 1 à 10, l'alcoxylate d'aminotriazine (a) étant préparé en l'absence d'un solvant.

13. Procédé pour la préparation d'alcoxylates d'aminotriazine selon l'une quelconque des revendications précédentes, la transformation de l'alcoxylate d'aminotriazine (a) avec au moins une aminotriazine (b) et au moins un oxyde d'alkylène (c) étant réalisée à des températures entre 100 et 200°C et à des pressions de 1-10 bars en l'absence d'un catalyseur et en l'absence d'un solvant.
